# EUROPÄISCHE PATENTSCHRIFT

(11) **EP 1 303 475 B1**
(45) Veröffentlichungstag und Bekanntmachung des Hinweises auf die Patenterteilung: **08.06.2005**
(21) Anmeldenummer: 01967196.5
(22) Anmeldetag: 17.07.2001
(51) Int. Cl.: C07C 67/31, C07C 69/716, C07C 69/675, C07D 303/42, C07D 339/04

(54) **VERFAHREN ZUR HERSTELLUNG VON ENANTIOMERENREINEN 6,8-DIHYDROXYOCTANSÄUREESTERN DURCH ASYMMETRISCHE KATALYTISCHE HYDRIERUNG**
METHOD FOR PRODUCING ENANTIOMER-FREE 6,8 DIHYDROXY OCTANOIC ACID ESTERS BY MEANS OF ASYMMETRIC, CATALYTIC HYDROGENATION
PROCEDE POUR LA PRODUCTION D'ESTERS DE L'ACIDE 6,8-DIHYDROCAPRYLIQUE EXEMPTS D'ENANTIOMERES PAR HYDROGENATION CATALYTIQUE ASYMETRIQUE

(30) Priorität: 27.07.2000 DE 10036516
(43) Veröffentlichungstag der Anmeldung: 23.04.2003
(73) Patentinhaber: VIATRIS GmbH & Co. KG, 61352 Bad Homburg (DE)
(72) Erfinder: GEWALD, Rainer, 01217 Dresden (DE)
(86) Internationale Anmeldenummer: PCT/EP2001/008244
(87) Internationale Veröffentlichungsnummer: WO 2002/010113

(56) Entgegenhaltungen:
- DE-A- 3 629 116
- DE-A- 19 709 069
- BEZBARUA, MAITREYEE SARMA ET AL: "A short enantioselective formal synthesis of methyl (S)-(-)-6,8-dihydroxyoctanoate. A key intermediate for the synthesis of R-(+)-.alpha.-lipoic acid" SYNTHESIS (1996), (11), 1289-1290 , XP002184344
- J. D. ELLIOTT ET AL.: "Asymmetric Synthesis via Acetal Templates: 12. Asymmetric Synthesis of R-(+)-.alpha.-Lipoic Acid" TETRAHEDRON LETTERS., Bd. 26, Nr. 21, 1985, Seiten 2535-2538, XP002184345 ELSEVIER SCIENCE PUBLISHERS, AMSTERDAM., NL ISSN: 0040-4039 in der Anmeldung erwähnt

## Beschreibung

### Technisches Gebiet

Die vorliegende Erfindung betrifft ein neues Verfahren zur Herstellung von enantiomerenreinen 6,8-Dihydroxyoctansäureestem der allgemeinen Formel I, wobei R¹ eine C₁-C₂₀-Alkylgruppe, C₃-C₁₂-Cycloalkylgruppe, C₇-C₁₂-Aralkylgruppe oder eine ein- oder zweikernige Arylgruppe bedeutet.

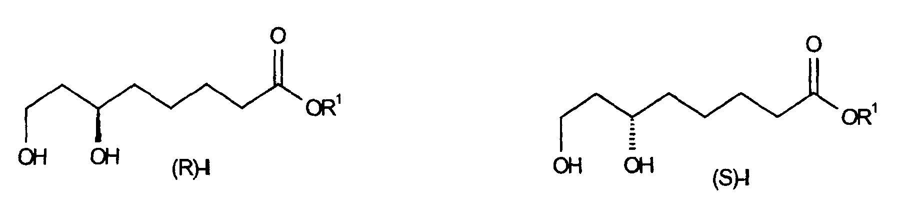

Des weiteren betrifft die Erfindung neue Verbindungen der Formeln II und III, die als Ausgangsverbindungen bzw. Zwischenprodukte bei der Synthese der Verbindungen (R)-I und (S)-I Verwendung finden.

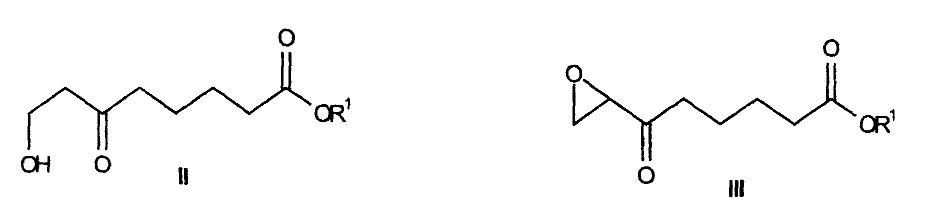

### Stand der Technik

Die Verbindungen (R)-I und (S)-I sind bekannt. Beide dienen vornehmlich als Zwischenprodukte für die Synthese von enantiomerenreiner α-Liponsäure der Formel IV und ihrer Derivate. α-Liponsäure ist 1,2-Dithiolan-3-pentansäure (Thioctsäure).

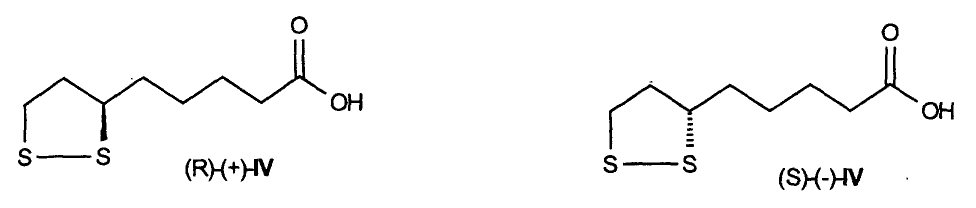

Das (R)-Enantiomer der α-Liponsäure (R)-(+)-IV ist ein Naturstoff, der in geringen Konzentrationen in praktisch allen tierischen und pflanzlichen Zellen vorkommt. Als Coenzym bei der oxidativen Decarboxylierung von α-Ketocarbonsäuren (z.B. Brenztraubensäure) ist α-Liponsäure von essentieller Bedeutung. α-Liponsäure ist pharmakologisch wirksam und weist antiphlogistische und antinociceptive (analgetische) sowie zytoprotektive Eigenschaften auf. Eine wichtige medizinische Indikation der razemischen α-Liponsäure ist die Behandlung der diabetischen Polyneuropathie. Nach neueren Ergebnissen (A. Baur et al., Klin. Wochenschr. 1991, 69, 722; J.P. Merin et al., FEBS Lett. 1996, 394, 9) kann α-Liponsäure möglicherweise Bedeutung bei der Bekämpfung durch HIV-1- und HTLV IIIB-Viren bedingter Krankheiten erlangen.

Bei den reinen optischen Isomeren der α-Liponsäure (R- und S-Form, d.h. (R)-α-Liponsäure und (S)-α-Liponsäure) ist im Gegensatz zu dem Razemat das (R)-Enantiomer vorwiegend antiphlogistisch und das (S)-Enantiomer vorwiegend antinociceptiv wirksam (EP 0427247, 08.11.90). Unterschiedliche pharmakokinetische Eigenschaften der beiden Enantiomere sind ebenfalls festgestellt worden (R. Hermann et al., Eur. J.Pharmaceut. Sci. 1996, 4, 167; G. Raddatz u. H. Bisswanger, J. Biotechnol. 1997, 58, 89; T.M. Hagen et al., FASEB J. 1999, 13, 411). Daher ist die Synthese der reinen Enantiomere von großer Wichtigkeit.

Bekannte Herstellungsverfahren der enantiomerenreinen α-Liponsäuren umfassen die Razematspaltung der α-Liponsäure oder ihrer Vorstufen, asymmetrische Synthesen unter Einsatz chiraler Auxilarien, "chiral pool"-Synthesen unter Verwendung von in der Natur vorkommender optisch aktiver Ausgangsverbindungen sowie mikrobielle Synthesen (Übersichtsartikel: J.S. Yadav et al., J. Sci. Ind. Res. 1990, 49, 400; sowie: E. Walton et al., J. Am. Chem. Soc. 1955, 77, 5144; D.S. Acker und W.J. Wayne, J. Am. Chem. Soc. 1957, 79, 6483; L.G. Chebotareva und A.M. Yurkevich, Khim.-Farm. Zh. 1980, 14, 92; A.S. Gopalan et al., Tetrahedron Lett. 1989, 5705; A.G. Tolstikov et al., Bioorg. Khim. 1990, 16, 1670; L. Dasaradhi et al., J. Chem. Soc., Chem. Commun. 1990, 729; A.S. Gopalan et al., J. Chem. Perkin Trans. 1 1990, 1897; EP 0487986 A2, 14.11.91; R. Bloch et al., Tetrahedron 1992, 48, 453; B. Adger et al., J. Chem. Soc., Chem. Commun. 1995, 1563; DE-OS 19533881.1, 13.09.95; DE-OS 19533882.1, 13.09.95; Y.R. Santosh Laxmi und D.S. lyengar, Synthesis, 1996, 594;; N.W. Fadnavis et al., Tetrahedron: Asymmetry 1997, 8, 337; N.W. Fadnavis et al., Tetrahedron: Asymmetry 1998, 9, 4109; S. Lee u. Y. Ahn, J. Korean Chem. Soc. 1999, 43, 128). In M. Bezbarua et al., Synthesis 1996, 1289 ist die enantioselektive Synthese von (S)-(-)-6,8-Dihydroxyoktansäuremethylester durch enzymatische Reduktion von Methyl-8-methoxy-6-oxooktanoat mittels Bäckerhefe und anschließende Methyletherspaltung beschrieben.

Davon stellt die Razematspaltung über die Bildung von diastereomeren Salzen der α-Liponsäure mit optisch aktivem α-Methylbenzylamin (DE-OS 4137773.7, 16.11.91 und DE-OS 4427079.8, 30.07.94) die bisher wirtschaftlichste Variante dar. Da die Razemattrennung erst auf der letzten Stufe der Synthesesequenz erfolgt, sind jedoch keine hohen Ausbeuten zu erzielen.

Die bekannten chemokatalytischen asymmetrischen Verfahren zur Herstellung von enantiomerenreiner α-Liponsäure (DE-OS 3629116.1, 27.08.86; DE-OS 19709069.1, 6.03.97; R. Zimmer et al., Tetrahedron: Asymmetry 2000, 11, 879) sind wegen der hohen Kosten der Ausgangsverbindungen unwirtschaftlich.

Aufgabe der Erfindung ist es deshalb, wahlweise die zu beiden Enantiomeren der α-Liponsäure führenden 6,8-Dihydroxyoctansäureester (R)-I und (S)-I in hoher chemischer und optischer Raum-Zeit-Ausbeute bei Verwendung kostengünstiger Ausgangsstoffe zugänglich zu machen.

### Beschreibung der Erfindung

Erfindungsgemäß gelingt dies durch asymmetrische chemokatalytische Hydrierung von 8-Hydroxy-6-oxo-octansäureestem der Formel II, bei der R¹ eine C₁-C₂₀-Alkylgruppe, C₃-C₁₂-Cycloalkylgruppe, C₇-C₁₂-Aralkylgruppe oder eine ein- oder zweikemige Arylgruppe bezeichnet, in Gegenwart von Komplexen aus Ruthenium und optisch aktiven Phosphinen.
Die Verbindungen II sind neu und können durch selektive Hydrierung der 7,8-Epoxy-6-oxo-octansäureester III, vorzugsweise in Gegenwart von Platin-, Palladium- oder Nickel-Katalysatoren, gewonnen werden.
Die Herstellung der ebenfalls neuen 7,8-Epoxy-6-oxo-octansäureester III gelingt in hohen Ausbeuten durch Epoxydierung von 6-Oxo-7-octensäureestem der Formel V, vorzugsweise mittels Natriumpercarbonat in Methanol.
Die Verbindungen V sind bekannt und sind durch Eliminierung von Chlorwasserstoff aus 8-Chlor-6-oxo-octansäureestern, die als kostengünstige Ausgangsverbindungen für die kommerzielle Synthese von razemischer α-Liponsäure verwendet werden, erhältlich (M.W. Bullock et al., J. Am. Chem. Soc. 1954, 76, 1828).

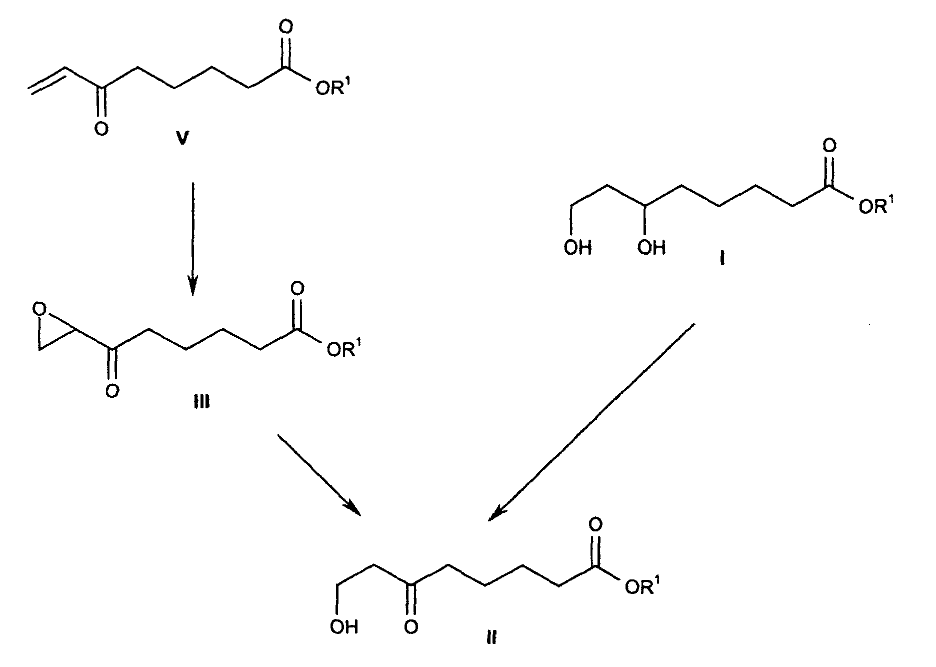

Alternativ lassen sich razemische 6,8-Dihydroxyoctansäureester der Formel I durch regioselektive Oxydation der sekundären Hydroxygruppe, vorzugsweise mittels Natriumhypochlorit in Essigsäure, in Verbindungen der Formel II überführen. Die Herstellung von razemischen 6,8-Dihydroxyoctansäureestern der Formel I ist bekannt und kann u.a. ausgehend von Butadien und Essigsäure erfolgen (J. Tsuji et al., J. Org. Chem. 1978, 43, 3606). Katalysatoren für die asymmetrische Hydrierung der Verbindungen II sind Ruthenium-Diphosphin-Komplexe der folgenden Formeln VI bis XII:

[RuHal₂D]ₙ(L)ₓ VI

[RuHalAD]⁺Y⁻ VII

RuDₙOOCR²OOCR³ VIII

[RuHₓDₙ]^{m+}Yₘ⁻ IX

[RuHal(PR⁴₂R⁵)D]²⁺Hal₂⁻ X

[RuHHalD₂] XI

[DRu(acac)₂] XII

worin:
- acac: für Acetylacetonat steht,
- D: für ein Diphosphin der allgemeinen Formel XIII steht,
- Hal: für Halogen, insbesondere Iod, Chlor oder Brom steht,
- R² und R³: gleich oder verschieden sind und für Alkyl mit bis zu 9 C-Atomen, vorzugsweise bis zu 4 C-Atomen, welches gegebenenfalls substituiert ist durch Halogen, insbesondere Fluor, Chlor oder Brom oder für Phenyl stehen, welches gegebenenfalls durch Alkyl mit 1 bis 4 C- Atomen substituiert ist oder für eine α- Aminoalkylsäure mit vorzugsweise bis zu 4 C-Atomen stehen, oder gemeinsam eine Alkylidengruppe mit bis zu 4 C-Atomen bilden,
- R⁴ und R⁵: jeweils gleich oder verschieden sind und für gegebenenfalls substituiertes Phenyl stehen, vorzugsweise substituiert durch Alkyl mit 1 bis 4 C-Atomen oder Halogen,
- Y: für Cl, Br, J, ClO₄, BF₄ oder PF₆ stehen,
- A: für einen unsubstituierten oder substituierten Benzolring wie p-Cymol steht,
- L: für einen neutralen Liganden wie Aceton, ein tertiäres Amin oder Dimethylformamid steht,
- n und m: jeweils für 1 oder 2 stehen,
- x: für 0 oder 1 steht,
wobei in Formel IX n für 1 und m für 2 steht, wenn x=0 bedeutet, und n für 2 und m für 1 steht, wenn x=1 bedeutet.

Die Komplexe der Formeln VI bis XII können nach an sich bekannten Methoden hergestellt werden (VI und XI: EP 174057 und J.P. Genet et al., Tetrahedron Asymmetry 1994, 5, 675; VII: EP 366390; VII: EP 245959 und EP 272787; IX: EP 256634; X: EP 470756; XII: P. Stahly et al., Organometallics 1993,1467).

Als optisch aktive Diphosphin-Liganden kommen Verbindungen der allgemeinen Formel XIII zur Anwendung: 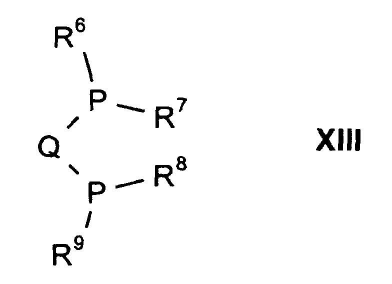 worin:
- Q: für eine die beiden P-Atome verbrückende Gruppe mit 2 bis 24 Kohlenstoffatomen und gegebenenfalls 1 bis 4 Heteroatomen, vorzugsweise O, S, N und Si, steht, wobei die Verbrückung von mindestens 2 der Kohlenstoffatome und gegebenenfalls 1 bis 4 der Heteroatome gebildet wird,
- R⁶- R⁹: jeweils gleich oder verschieden sind und für Alkylgruppen mit 1 bis 18 C-Atomen, Cycloalkylgruppen mit 5 bis 7 C-Atomen oder Arylgruppen mit 6 bis 12 C-Atomen stehen.

Als besonders bevorzugte, in enantiomerenreiner Form zum Einsatz kommende chirale Diphosphine können folgende Liganden als Beispiele aufgeführt werden:

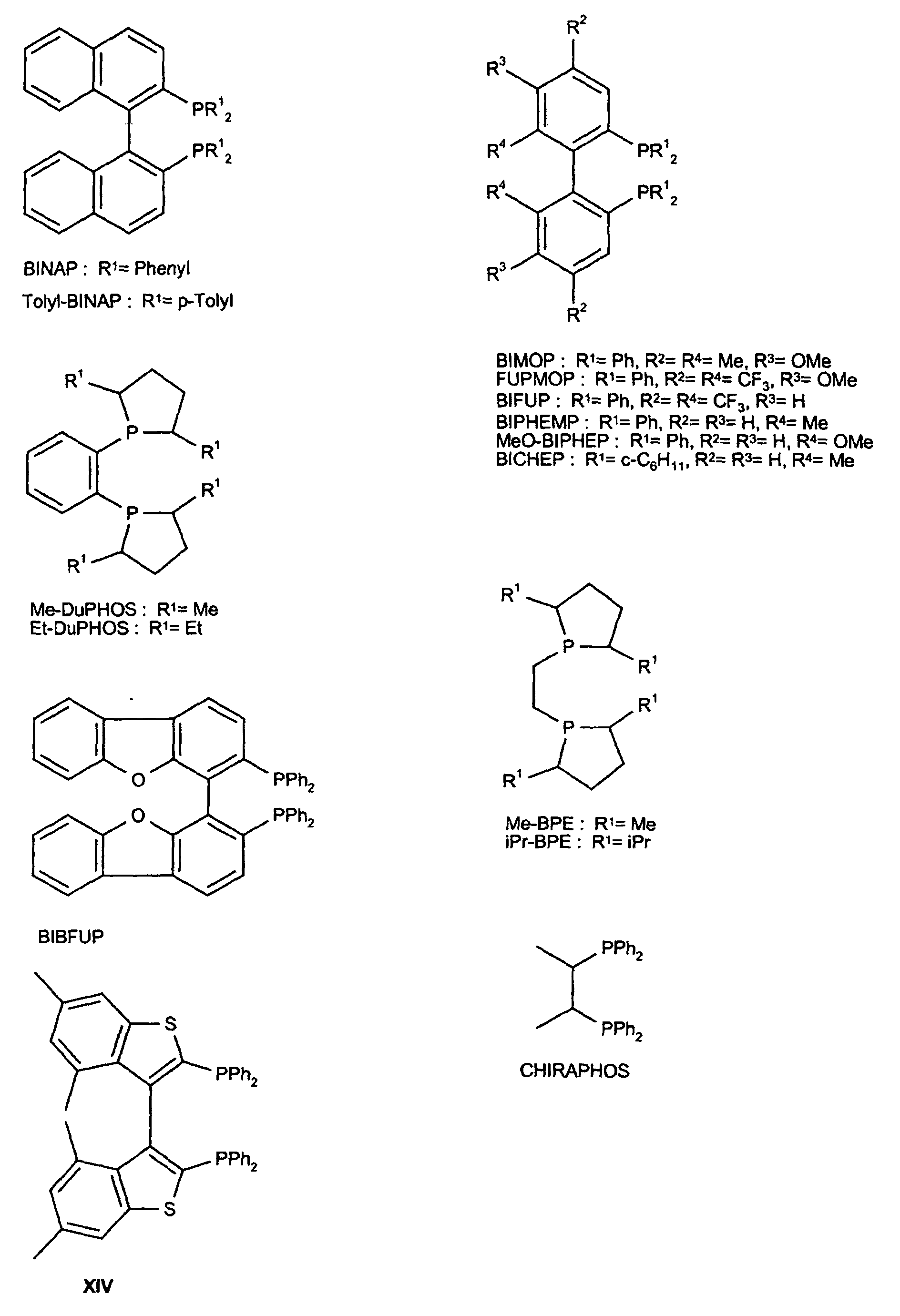

Die oben der Einfachheit halber als razemische Strukturen aufgeführten Liganden sind in ihren enantiomerenreinen Formen bekannte Verbindungen (BINAP: R. Noyori et al., J. Am. Chem. Soc. 1980, 102, 7932; BIMOP, FUPMOP, BIFUP: M. Murata et al., Synlett 1991,827; BIBHEMP: R. Schmid et al., Helv. Chim. Acta 1988, 71, 697; MeO-BIPHEP: R. Schmid et al., Helv. Chim. Acta 1991, 74, 370; BICHEP: A. Miyashita et al., Chem. Lett. 1989, 1849; DuPHOS: M. Burk et al., Organometallics 1990, 9, 2653; BPE: M. Burk et al., J. Am. Chem. Soc. 1995, 117, 4423; BIBFUP: EP 643065; CHIRAPHOS: B. Bosnich et al., J. Am. Chem. Soc. 1977, 99, 6262; XIV: WO 96/01831).

Die asymmetrische Hydrierung der Verbindungen der Formel II in Gegenwart der oben beschriebenen optisch aktiven Ruthenium-Diphosphin-Komplexe der Formeln VI bis XII kann in geeigneten, unter den Reaktionsbedingungen inerten organischen Lösungsmitteln durchgeführt werden. Als solche können insbesondere genannt werden, Alkohole wie Methanol oder Ethanol, chlorierte Kohlenwasserstoffe wie Methylenchlorid oder Dichlorethan, cyclische Ether wie Tetrahydrofuran oder Dioxan, Ester wie z.B. Essigester, aromatische Kohlenwasserstoffe wie Benzol oder Toluol, oder auch Gemische hiervon und dergleichen. Zur Unterdrückung einer möglichen Ketalbildung beim Arbeiten in Alkoholen als Lösungsmittel können bis zu 10 Vol.-% Wasser zugesetzt werden. Die Substratkonzentrationen liegen vorzugsweise bei 5 bis 50 Vol.-%, insbesondere bei 20 bis 40 Vol.-%.

Die Umsetzungen können vorzugsweise bei Temperaturen von etwa 10°C bis 140°C, insbesondere von etwa 20°C bis 70°C und unter einem Wasserstoffdruck von etwa 1 bis 100 bar, insbesondere von 4 bis 50 bar, durchgeführt werden. Die Reaktionszeiten betragen im allgemeinen 2 bis 48 Stunden, meistens 6 bis 24 Stunden. Das molare Verhältnis zwischen Ruthenium in den Komplexen VI bis XII und den zu hydrierenden Verbindungen II liegt zweckmäßig zwischen etwa 0,001 und etwa 5 Mol.-%, vorzugsweise zwischen etwa 0,005 und etwa 0,2 Mol.-%.

In der Reaktion kann das gewünschte Enantiomer der Formel I durch Auswahl des optisch aktiven Diphosphinliganden der Formel XIII mit der entsprechenden Konfiguration erhalten werden. So führt beispielsweise die Verwendung von (R)-(+)-BINAP zu Produkten der Formel (R)-I, der Einsatz von (S)-(-)-BINAP zu Produkten der Formel (S)-I.

Die Verbindungen (S)-I und(R)-I dienen zur Herstellung der enantiomerenreinen α-Liponsäuren der Formel IV, indem diese auf bekanntem Wege (J.D. Gopalan et al., Tetrahedron Lett. 1985, 2535):
a) in organischer Lösung mit einem Sulfonsäurechlorid und einer tertiären Stickstoffbase in den Bissulfonsäureester von I überführt werden,
b) diese Verbindung in einem polaren Lösungsmittel mit Schwefel und einem Alkalimetallsulfid zum α-Liponsäureester umgesetzt wird und
c) dieser Ester gewünschtenfalls in das jeweilige reine Enantiomer der α-Liponsäure überführt wird. Dabei wird ausgehend von den Verbindungen (R)-I die (S)-(-)- α-Liponsäure und ausgehend von den Verbindungen (S)-I die (R)-(+)-α-Liponsäure erhalten.

Die nach dem erfindungsgemäßen Verfahren hergestellten Verbindungen (R)-I und (S)-I sowie (R)-(+)-IV und die (S)-(-)-IV weisen in der Regel einen hohen Enantiomerenüberschuß auf, entsprechend einer optischen Ausbeute von 90 bis 99%.

Die Enantiomerenverhältnisse werden direkt durch chirale HPLC oder GC an optisch aktiven Säulen gemessen.

Die vorliegende Erfindung ermöglicht es, die enantiomerenreinen
6,8-Dihydroxyoctansäureester der allgemeinen Formel I (R¹ = C₁-C₂₀-Alkyl, C₃-C₁₂-Cycloalkyl, C₇-C₁₂-Aralkyl oder ein- oder zweikemiges Aryl) als Zwischenprodukte zur Herstellung der enantiomerenreinen α-Liponsäuren der Formel IV auf wirtschaftliche Weise in hohen chemischen und optischen Ausbeuten zugänglich zu machen.

Die nachfolgenden Beispiele erläutern die Erfindung, ohne diese zu beschränken.

### Beispiel 1

In ein 20 ml-Schlenkgefäß wurden unter Argon 43,5 mg (0,087 mmol) [RuCl₂(C₆H₆)]₂, 113,7 mg (0,183 mmol) (R)-BINAP und 3 ml Dimethylformamid gegeben. Die rötlichbraune Suspension wurde 10 min auf 100°C erhitzt. Die nunmehr klare Lösung wurde abgekühlt und im Vakuum (1 bis 0,1 mmHg) bei 50°C unter starkem Rühren über einen Zeitraum von 1 h eingeengt. Der verbliebene orangebraune Feststoff wurde in 1 ml Tetrahydrofuran aufgenommen und kam so als Ru-(R)-BINAP-Katalysator in den asymmetrischen Hydrierungen zum Einsatz.

### Beispiel 2

In ein 20 ml-Schlenkgefäß wurden unter Argon 43,5 mg (0,087 mmol) [RuCl₂(C₆H₆)]₂, 113,7 mg (0,183 mmol) (S)-BINAP und 3 ml Dimethylformamid gegeben. Die rötlichbraune Suspension wurde 10 min auf 100°C erhitzt. Die nunmehr klare Lösung wurde abgekühlt und im Vakuum (1 bis 0,1 mmHg) bei 50°C unter starkem Rühren über einen Zeitraum von 1 h eingeengt. Der verbliebene orangebraune Feststoff wurde in 1 ml Tetrahydrofuran aufgenommen und kam so als Ru-(S)-BINAP-Katalysator in den asymmetrischen Hydrierungen zum Einsatz.

### Beispiel 3

Ein 100 ml-Autoklav wurde unter Argon mit 3,8 g (20 mmol) 8-Hydroxy-6-oxooctansäuremethylester, mit der unter Beispiel 1 hergestellten Ru-(R)-BINAP-Katalysatorlösung und mit 20 ml sauerstofffreiem Methanol beladen. Die Hydrierung wurde bei 60°C, einem konstanten Druck von 40 bar reinem H₂ und unter intensivem Rühren 20 Stunden durchgeführt. Nach Beendigung der Reaktion wurde das Lösungsmittel am Rotationsverdampfer abdestilliert. Man erhielt nach Reinigung des Rückstands durch Säulenchromatographie (Kieselgel, Ethylacetat/n-Hexan) 3,2 g (85%) (R)-6,8-Dihydroxyoctansäuremethylester mit einem Enantiomerenüberschuß von 96% (chirale GC).

### Beispiel 4

Ein 100 ml-Autoklav wurde unter Argon mit 3,8 g (20 mmol) 8-Hydroxy-6-oxooctansäuremethylester, mit der unter Beispiel 2 hergestellten Ru-(S)-BINAP-Katalysatorlösung und mit 20 ml sauerstofffreiem Methanol beladen. Die Hydrierung wurde bei 60°C, einem konstanten Druck von 40 bar reinem H₂ und unter intensivem Rühren 20 Stunden durchgeführt. Nach Beendigung der Reaktion wurde das Lösungsmittel am Rotationsverdampfer abdestilliert. Man erhielt nach Reinigung des Rückstands durch Säulenchromatographie (Kieselgel, Ethylacetat/n-Hexan) 3,1 g (82%) (S)-6,8-Dihydroxyoctansäuremethylester mit einem Enantiomerenüberschuß von 96% (chirale GC).

### Beispiel 5

Zu 16,6 g (87 mmol) 6,8-Dihydroxyoctansäuremethylester in 200 ml Eisessig wurden bei Raumtemperatur 100 ml wässrige Natriumhypochlorit-Lösung (10-13% aktives Chlor) über einen Zeitraum von 45 Minuten zugetropft. Nach weiteren 3 Stunden Rühren bei Raumtemperatur wurden zur Vernichtung von überschüssigem Natriumhypochlorit 180 ml Isopropanol zugefügt und 10 Minuten gerührt. Anschließend wurde das Reaktionsgemisch in 1200 ml Wasser gegeben und mehrmals mit Methylenchlorid extrahiert. Die vereinigten organischen Phasen wurden mit kaltgesättigter Natriumhydrogencarbonat-Lösung gewaschen. Nach Trocknen über Natriumsulfat wurde das Lösungsmittel am Rotationsverdampfer abdestilliert. Man erhielt 13,0 g (80%) 8-Hydroxy-6-oxo-octansäuremethylester.
¹³C NMR (CDCl₃): δ = 23.4, 25.3, 34.0, 42.8, 45.2, 51.7, 57.9, 174.1, 211.0

### Beispiel 6

Ein 100 ml-Autoklav wurde unter Argon mit 9,4 g (50 mmol) 7,8-Epoxy-6-oxooctansäuremethylester, mit 0,4 g Platin(IV)-oxid-Katalysator und mit 50 ml Ethylacetat beladen. Die Hydrierung wurde bei 20°C, einem konstanten Druck von 50 bar reinem H₂ und unter intensivem Rühren 16 Stunden durchgeführt. Nach Beendigung der Reaktion wurde der Katalysator abfiltriert und das Lösungsmittel am Rotationsverdampfer abdestilliert. Man erhielt nach Reinigung des Rückstands durch Säulenchromatographie (Kieselgel, Ethylacetat/n-Hexan) 6,3 g (67%) 8-Hydroxy-6-oxo-octansäuremethylester.

### Beispiel 7

Zu 13,9 g (82 mmol) 6-Oxo-7-octensäuremethylester in 210 ml Methanol wurden unter Rühren bei Raumtemperatur 39,1 g (250 mmol) Natriumpercarbonat in vier Portionen über einen Zeitraum von 2 Stunden zugegeben. Nach einer weiteren Stunde Rühren bei Raumtemperatur wurde das Reaktionsgemisch in 1000 ml Wasser gegeben und mehrmals mit Methylenchlorid extrahiert. Die vereinigten organischen Phasen wurden mit Wasser gewaschen. Nach Trocknen über Natriumsulfat wurde das Lösungsmittel am Rotationsverdampfer abdestilliert. Man erhielt 13,5 g (88%) 7,8-Epoxy-6-oxo-octansäuremethylester.
¹³C NMR (CDCl₃): δ = 21.8, 23.2, 32.4, 41.5, 50.1, 57.4, 66.5, 172.5, 207.4

## Patentansprüche

1. Verfahren zur Herstellung von Verbindungen der allgemeinen Formel I 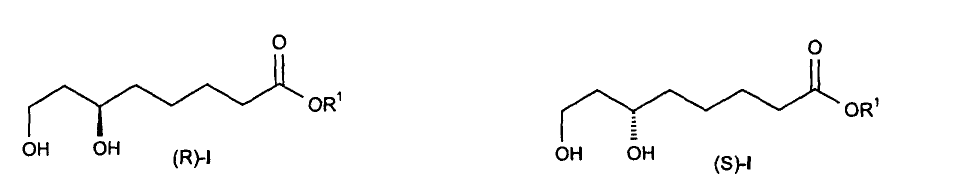 in der R¹ eine C₁-C₂₀-Alkylgruppe,
C₃-C₁₂-Cycloalkylgruppe, C₇-C₁₂-Aralkylgruppe oder eine ein- oder zweikemige Arylgruppe bedeutet,
**dadurch gekennzeichnet, dass** man ein Keton der Formel II 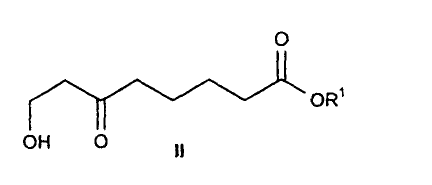 in der R¹ die obige Bedeutung hat,
asymmetrisch in Gegenwart eines Ruthenium-Diphosphin-Komplexes der Formeln VI bis XII hydriert.
[RuHal₂D]ₙ(L)ₓ VI
[RuHalAD]⁺Y⁻ VII
RuDₙOOCR²OOCR³ VIII
[RuHₓDₙ]^{m+}Yₘ⁻ IX
[RuHal(PR⁴ ₂R⁵)D]²⁺Hal₂⁻ X
[RuHHalD₂] XI
[DRu(acac)₂] XII
worin:
acac für Acetylacetonat steht,
D für ein Diphosphin der allgemeinen Formel XIII steht,
Hal für Halogen steht,
R² und R³ gleich oder verschieden sind und für Alkyl mit bis zu 9 C-Atomen, welches gegebenenfalls substituiert ist durch Halogen, oder für Phenyl stehen, welches gegebenenfalls durch Alkyl mit 1 bis 4 C- Atomen substituiert ist oder für eine α- Aminoalkylsäure stehen, oder gemeinsam eine Alkylidengruppe mit bis zu 4 C-Atomen bilden,
R⁴ und R⁵ jeweils gleich oder verschieden sind und für gegebenenfalls substituiertes Phenyl stehen,
Y für Cl, Br, J, ClO₄, BF₄ oder PF₆ stehen,
A für einen unsubstituierten oder substituierten Benzolring wie p-Cymol steht,
L für einen neutralen Liganden wie Aceton, ein tertiäres Amin oder Dimethylformamid steht,
n und m jeweils für 1 oder 2 stehen,
x für 0 oder 1 steht,
wobei in Formel IX n für 1 und m für 2 steht, wenn x=0 bedeutet, und n für 2 und m für 1 steht, wenn x=1 bedeutet.
und als optisch aktive Diphosphin-Liganden D Verbindungen der allgemeinen Formel XIII 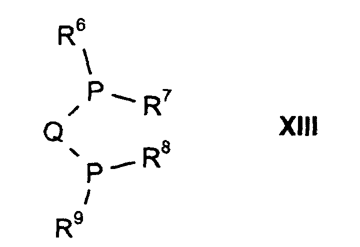 worin:
Q für eine die beiden P-Atome verbrückende Gruppe mit 2 bis 24 Kohlenstoffatomen und gegebenenfalls 1 bis 4 Heteroatomen, steht, wobei die Verbrückung von mindestens 2 der Kohlenstoffatome und gegebenenfalls 1 bis 4 der Heteroatome gebildet wird,
R⁶- R⁹ jeweils gleich oder verschieden sind und für Alkylgruppen mit 1 bis 18 C-Atomen, Cycloalkylgruppen mit 5 bis 7 C-Atomen oder Arylgruppen mit 6 bis 12 C-Atomen stehen.
zur Anwendung kommen.

2. Verfahren gemäß Anspruch 1, **dadurch gekennzeichnet, dass**
in den Ruthenium-Diphosphin-Komplexen der Formeln VI bis XII
Hal für Iod, Chlor oder Brom steht

3. Verfahren gemäß Anspruch 1, **dadurch gekennzeichnet, dass**
in den Ruthenium-Diphosphin-Komplexen der Formeln VI bis XII
R² und R³ gleich oder verschieden sind und für Alkyl mit vorzugsweise bis zu 4 C-Atomen, welches gegebenenfalls substituiert ist durch Fluor, Chlor oder Brom oder für Phenyl stehen, welches gegebenenfalls durch Alkyl mit 1 bis 4 C-Atomen substituiert ist oder für eine α- Aminoalkylsäure mit vorzugsweise bis zu 4 C-Atomen stehen, oder gemeinsam eine Alkylidengruppe mit bis zu 4 C-Atomen bilden

4. Verfahren gemäß Anspruch 1, **dadurch gekennzeichnet, dass**
in den Ruthenium-Diphosphin-Komplexen der Formeln VI bis XII
R⁴ und R⁵ jeweils gleich oder verschieden sind und für durch Alkyl mit 1 bis 4 C-Atomen oder Halogen substituiertes Phenyl stehen.

5. Verfahren gemäß Anspruch 1, **dadurch gekennzeichnet, dass** in dem optisch aktiven Diphosphin-Liganden D der allgemeinen Formel XIII
Q für eine die beiden P-Atome verbrückende Gruppe mit 2 bis 24 Kohlenstoffatomen und gegebenenfalls 1 bis 4 Heteroatomen, ausgewählt aus der Gruppe von O, S, N und Si, steht, wobei die Verbrückung von mindestens 2 der Kohlenstoffatome und gegebenenfalls 1 bis 4 der Heteroatome gebildet wird

6. Verfahren gemäß einem der Ansprüche 1 bis 5, **dadurch gekennzeichnet, dass** man die asymmetrische Hydrierung bei Temperaturen von etwa 10°C bis etwa 140°C und unter einem Druck von etwa 1 bis 100 bar durchführt.

7. Verfahren gemäß einem der Ansprüche 1 bis 5, **dadurch gekennzeichnet, dass** man die asymmetrische Hydrierung bei Reaktionszeiten von 2 bis 48 Stunden bei einem molaren Verhältnis zwischen Ruthenium in den Komplexen VI bis XII und den zu hydrierenden Verbindungen II zwischen etwa 0,001 und etwa 5 Mol% durchführt.

8. 8-Hydroxy-6-oxo-octansäureester der allgemeinen Formel II 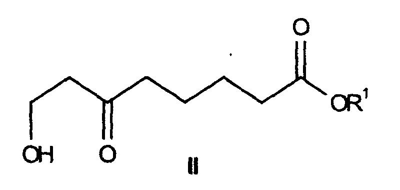 in der R¹ eine C₁-C₂₀-Alkylgruppe,
C₃-C₁₂-Cycloalkylgruppe, C₇-C₁₂-Aralkylgruppe oder eine ein- oder zweikemige Arylgruppen bezeichnet.

9. 7,8-Epoxy-6-oxo-octansäureester der allgemeinen Formel III 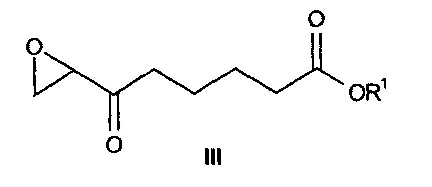 in der R¹ eine C₁-C₂₀-Alkylgruppe,
C₃-C₁₂-Cycloalkylgruppe, C₇-C₁₂-Aralkylgruppe oder eine ein- oder zweikemige Arylgruppen bezeichnet.

10. Verfahren zur Herstellung von (R)-(+)- α-Liponsäure der Formel (R)-(+)-IV 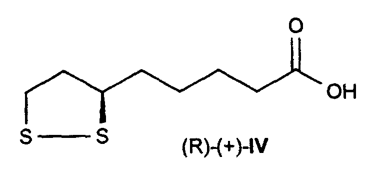 **dadurch gekennzeichnet, dass** man die Verbindungen II durch ein Verfahren gemäß Anspruch 1 asymmetrisch zu den Verbindungen (S)-I hydriert und diese auf bekanntem Weg
a) in organischer Lösung mit einem Sulfonsäurechlorid und einer tertiären Stickstoffbase in den Bissulfonsäureester von (S)-I überführt,
b) die in Schritt a) erhaltene Verbindung in einem polaren Lösungsmittel mit Schwefel und einem Alkalimetallsulfid zum (R)-(+)- α-Liponsäureester umsetzt und
c) dieser Ester in die (R)-(+)- α-Liponsäure überführt wird.

11. Verfahren zur Herstellung von (S)-(-)- α-Liponsäure der Formel (S)-(-)-IV 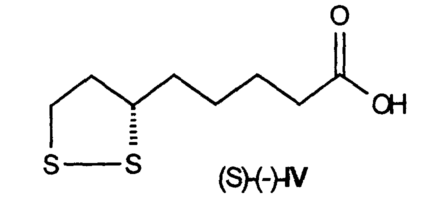 **dadurch gekennzeichnet, dass** man die Verbindungen II durch ein Verfahren gemäß Anspruch 1 asymmetrisch zu den Verbindungen (R)-I hydriert und diese auf bekanntem Weg
a) in organischer Lösung mit einem Sulfonsäurechlorid und einer tertiären Stickstoffbase in den Bissulfonsäureester von (R)-I überführt,
b) die in Schritt a) erhaltene Verbindung in einem polaren Lösungsmittel mit Schwefel und einem Alkalimetallsulfid zum (S)-(-)-α-Liponsäureester umsetzt und
c) dieser Ester in die (S)-(-)-α-Liponsäure überführt wird.

## Claims

1. Process for preparing compounds of the general formula I 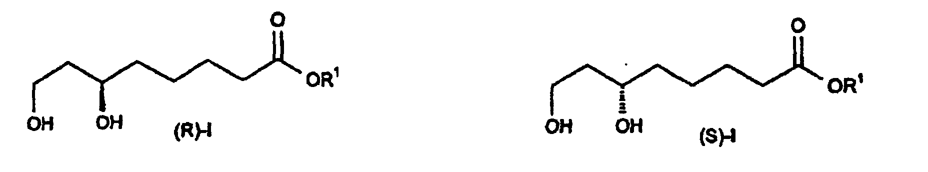 in which R¹ is a C₁-C₂₀-alkyl group, C₃-C₁₂-cycloalkyl group, C₇-C₁₂-aralkyl group or a mono- or bicyclic aryl group,
**characterized in that** a ketone of the formula II 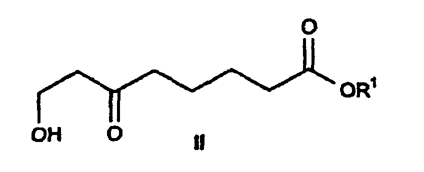 in which R¹ is as defined above
is hydrogenated asymmetrically in the presence of a ruthenium-diphosphine complex of the formulae VI to XII
[RuHal₂D]ₙ(L)ₓ VI
[RuHalAD]⁺Y⁻ VII
RuDₙOOCR²OOCR³ VIII
[RuHₓDₙ]^{m+}Yₘ⁻ IX
[RuHal (PR⁴ ₂R⁵)D]²⁺Hal₂⁻ X
[RuHHalD₂] XI
[DRu(acac)₂] XII
in which:
acac is acetylacetonate,
D is a diphosphine of the general formula XIII,
Hal is halogen,
R² and R³ are the same or different and are alkyl having up to 9 carbon atoms which is optionally substituted by halogen, or are phenyl which is optionally substituted by alkyl having 1 to 4 carbon atoms, or are an α-aminoalkyl acid, or together form an alkylidene group having up to 4 carbon atoms,
R⁴ and R⁵ are each the same or different and are optionally substituted phenyl,
Y is Cl, Br, I, ClO₄, BF₄ or PF₆,
A is an unsubstituted or substituted benzene ring as p-cymene,
L is an uncharged ligand such as acetone, a tertiary amine or dimethylformamide,
n and m are each 1 or 2,
x is 0 or 1,
where, in formula IX, n is 1 and m is 2 when x = 0, and n is 2 and m is 1 when x = 1,
and the optical active diphosphine ligands D used are compounds of the general formula XIII 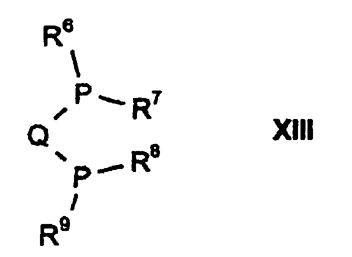 in which:
Q is a group which joins the two phosphorus atoms and has 2 to 24 carbon atoms and optionally 1 to 4 heteroatoms, the joining group being formed by at least 2 of the carbon atoms and optionally 1 to 4 of the heteroatoms,
R⁶-R⁹ are each the same or different and are alkyl groups having 1 to 18 carbon atoms, cycloalkyl groups having 5 to 7 carbon atoms or aryl groups having 6 to 12 carbon atoms.

2. Process according to Claim 1, **characterized in that**, in the ruthenium-diphosphine complexes of the formulae VI to XII,
Hal is iodine, chlorine or bromine.

3. Process according to Claim 1, **characterized in that**,
in the ruthenium-diphosphine complexes of the formulae VI to XII,
R² and R³ are the same or different and are each alkyl having preferably up to 4 carbon atoms which is optionally substituted by fluorine, chlorine or bromine, or are phenyl which is optionally substituted by alkyl having 1 to 4 carbon atoms, or are an α-aminoalkyl acid having preferably up to 4 carbon atoms, or together form an alkylidene group having up to 4 carbon atoms.

4. Process according to Claim 1, **characterized in that**,
in the ruthenium-diphosphine complexes of the formulae VI to XII,
R⁴ and R⁵ are each the same or different and are phenyl substituted by alkyl having 1 to 4 carbon atoms or halogen.

5. Process according to Claim 1, **characterized in that**,
in the optically active diphosphine ligand D of the general formula XIII,
Q is a group which joins the two phosphorus atoms and has 2 to 24 carbon atoms and optionally 1 to 4 heteroatoms selected from the group of O, S, N and Si, the joining group being formed by at least 2 of the carbon atoms and optionally 1 to 4 of the heteroatoms.

6. Process according to one of Claims 1 to 5,
**characterized in that** the asymmetric hydrogenation is carried out at temperatures of about 10°C to about 140°C and under a pressure of about 1 to 100 bar.

7. Process according to one of Claims 1 to 5,
**characterized in that** the asymmetric hydrogenation is carried out at reaction times of 2 to 48 hours at a molar ratio between ruthenium in the complexes VI to XII and the compounds II to be hydrogenated between about 0.001 and about 5 mol%.

8. 8-Hydroxy-6-oxooctanoic esters of the general formula II 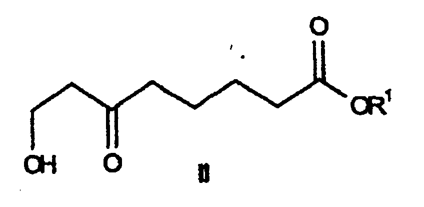 in which R¹ is a C₁-C₂₀-alkyl group, C₃-C₁₂-cycloalkyl group, C₇-C₁₂-aralkyl group or a mono- or bicyclic aryl group.

9. 7,8-Epoxy-6-oxooctanoic esters of the general formula III 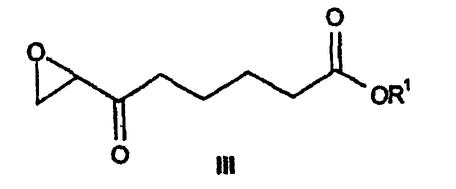 in which R¹ is a C₁-C₂₀-alkyl group, C₃-C₁₂-cycloalkyl group, C₇-C₁₂-aralkyl group or a mono- or bicyclic aryl group.

10. Process for preparing (R)-(+)-α-lipoic acid of the formula (R)-(+)-IV 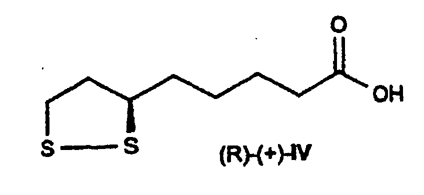 **characterized in that** the compounds II are hydrogenated asymmetrically to give the compounds (S)-I by a process according to Claim 1, and the latter compounds, by a known route,
a) are converted in organic solution using a sulphonyl chloride and a tertiary nitrogen base to the bissulphonic ester of (S)-I,
b) the compound obtained in step a) in a polar solvent is reacted with sulphur and an alkali metal sulphide to give (R)-(+)-α-lipoic ester and
c) this ester is converted to (R)-(+)-α-lipoic acid.

11. Process for preparing (S)-(-)-α-lipoic acid of the formula (S)-(-)-IV 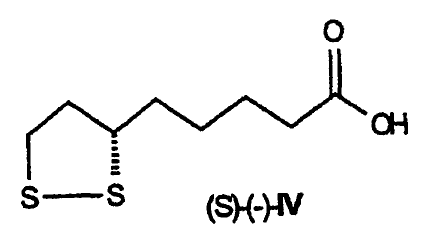 **characterized in that** the compounds II are hydrogenated asymmetrically to give the compounds (R)-I by a process according to Claim 1, and the latter compounds, by a known route,
a) are converted in organic solution using a sulphonyl chloride and a tertiary nitrogen base to the bissulphonic ester of (R)-I,
b) the compound obtained in step a) in a polar solvent is reacted with sulphur and an alkali metal sulphide to give (S)-(-)-α-lipoic ester and
c) this ester is converted to (S)-(-)-α-lipoic acid.

## Revendications

1. Procédé pour la préparation de composés de formule générale I 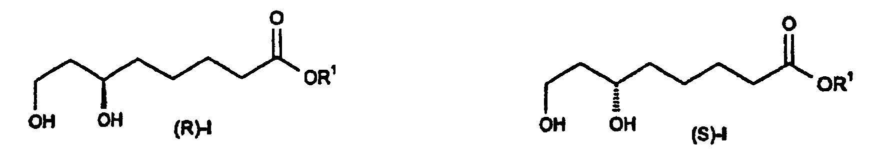 dans laquelle R¹ représente un groupe alkyle en C₁ à C₂₀, un groupe cycloalkyle en C₃ à C₁₂, un groupe aralkyle en C₇ à C₁₂ ou un groupe aryle mono- ou dinucléaire, **caractérisé en ce que** l'on réalise l'hydrogénation d'une cétone de formule II 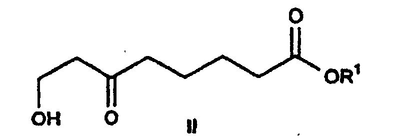 dans laquelle R¹ a la signification ci-dessus,
de manière asymétrique en présence d'un complexe de diphosphine de ruthénium des formules VI à XII.
[RuHal₂D]ₙ(L)ₓ VI
[RuHalAD]⁺Y⁻ VII
RuDₙOOCR²OOCR³ VIII
[RuHₓDₙ]^{m+}Yₘ⁻ IX
[RuHal(PR⁴ ₂R⁵)D]²⁺Hal₂⁻ X
[RuHHalD₂] XI
[DRu(acac)₂] XII
dans lesquelles :
acac représente un acétylacétonate,
D représente une diphosphine de formule générale XIII,
Hal représente un halogène,
R² et R³ sont identiques ou différents et représentent un alkyle avec jusqu'à 9 atomes de carbone, qui est éventuellement substitué par un halogène, ou un phényle, qui est éventuellement substitué par un alkyle avec de 1 à 4 atomes de carbone ou représentent un acide α-aminoalkylique, ou forment ensemble un groupe alkylidène avec jusqu'à 4 atomes de carbone,
R⁴ et R⁵ sont respectivement identiques ou différents et représentent un phényle éventuellement substitué,
Y représente Cl, Br, I, ClO₄, BF₄ ou PF₆,
A représente un cycle benzène non substitué ou substitué comme le p-cymène,
L représente un ligand neutre comme l'acétone, une amine tertiaire ou le diméthylformamide,
n et m représentent respectivement 1 ou 2,
x représente 0 ou 1,
dans la formule IX, n représentant 1 et m 2, lorsque x = 0, et n représentant 2 et m 1, lorsque x = 1,
et **en ce que** l'on utilise comme ligands diphosphine optiquement actifs D des composés de formule générale XIII 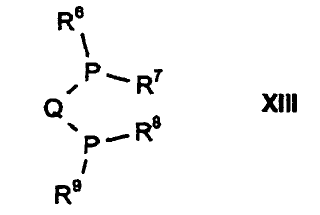 dans laquelle :
Q représente un groupe pontant les deux atomes P avec de 2 à 24 atomes de carbone et éventuellement de 1 à 4 hétéroatomes, le pont étant formé d'au moins 2 des atomes de carbone et éventuellement de 1 à 4 des hétéroatomes,
R⁶ à R⁹ sont respectivement identiques ou différents et représentent des groupes alkyle avec de 1 à 18 atomes de carbone, des groupes cycloalkyle avec de 5 à 7 atomes de carbone ou des groupes aryle avec de 6 à 12 atomes de carbone.

2. Procédé selon la revendication 1, **caractérisé en ce que** dans les complexes de diphosphine de ruthénium des formules VI à XII
Hal représente l'iode, le chlore ou le brome.

3. Procédé selon la revendication 1, **caractérisé en ce que** dans les complexes de diphosphine de ruthénium des formules VI à XII
R² et R³ sont identiques ou différents et représentent un alkyle avec de préférence jusqu'à 4 atomes de carbone, qui est éventuellement substitué par un fluor, chlore ou brome, ou un phényle, qui est éventuellement substitué par un alkyle avec de 1 à 4 atomes de carbone ou représentent un acide α-aminoalkylique avec de préférence jusqu'à 4 atomes de carbone, ou forment ensemble un groupe alkylidène avec jusqu'à 4 atomes de carbone.

4. Procédé selon la revendication 1, **caractérisé en ce que** dans les complexes de diphosphine de ruthénium des formules VI à XII
R⁴ et R⁵ sont respectivement identiques ou différents et représentent un phényle substitué par un alkyle avec de 1 à 4 atomes de carbone ou un halogène.

5. Procédé selon la revendication 1, **caractérisé en ce que** dans le ligand de diphosphine optiquement actif D de formule générale XIII
Q représente un groupe pontant les 2 atomes P avec de 2 à 24 atomes de carbone et éventuellement de 1 à 4 hétéroatomes, choisis dans le groupe de O, S, N et Si, le pont étant formé par au moins 2 des atomes de carbone et éventuellement 1 à 4 des hétéroatomes.

6. Procédé selon l'une quelconque des revendications 1 à 5, **caractérisé en ce que** l'on réalise l'hydrogénation asymétrique à des températures d'environ 10°C jusqu'à environ 140°C et sous une pression d'environ 1 à 100 bar.

7. Procédé selon l'une quelconque des revendications 1 à 5, **caractérisé en ce que** l'on réalise l'hydrogénation asymétrique à des temps de réaction de 2 à 48 heures pour un rapport molaire entre le ruthénium dans les complexes VI à XII et les composés hydrogénants II entre environ 0,001 et environ 5% en moles.

8. Ester de l'acide 8-hydroxy-6-oxo-octanoïque de formule générale II 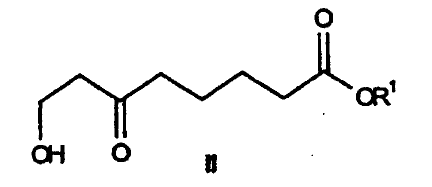 dans laquelle R¹ représente un groupe alkyle en C₁ à C₂₀, un groupe cycloalkyle en C₃ à C₁₂, un groupe aralkyle en C₇ à C₁₂ ou un groupe aryle mono- ou binucléaire.

9. Ester de l'acide 7,8-époxy-6-oxo-octanoique de formule générale III 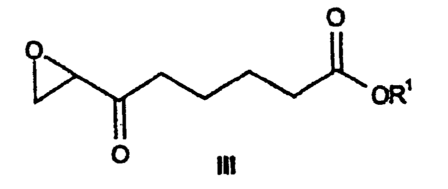 dans laquelle R¹ représente un groupe alkyle en C₁ à C₂₀, un groupe cycloalkyle en C₃ à C₁₂, un groupe aralkyle en C₇ à C₁₂ ou un groupe aryle mono- ou binucléaire.

10. Procédé pour la production d'acide (R)-(+)-α-lipoïque de formule (R)-(+)-IV 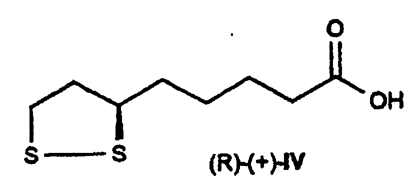 **caractérisé en ce que** l'on hydrogène les composés II au moyen d'un procédé selon la revendication 1 de manière asymétrique pour obtenir les composés (S)-I et **en ce que** l'on effectue de manière connue
a) la transformation de ceux-ci en solution organique avec un chlorure d'acide sulfonique et une base azotée tertiaire en ester d'acide bissulfonique de (S)-I,
b) la réaction du composé obtenu à l'étape a) dans un solvant polaire avec du soufre et un sulfure de métal alcalin pour obtenir l'ester d'acide (R)-(+)-α-lipoïque et
c) la transformation de cet ester en acide (R)-(+)-α-lipoïque.

11. Procédé pour la production d'acide (S)-(-)-α-lipoique de formule (S)-(-)-IV 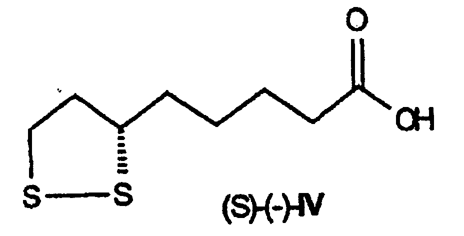 **caractérisé en ce que** l'on hydrogène les composés II au moyen d'un procédé selon la revendication 1 de manière asymétrique pour obtenir les composés (R)-I et **en ce que** l'on effectue de manière connue
a) la transformation de ceux-ci en solution organique avec un chlorure d'acide sulfonique et une base azotée tertiaire pour obtenir l'ester d'acide bissulfonique de (R)-I,
b) la réaction du composé obtenu à l'étape a) dans un solvant polaire avec du soufre et un sulfure de métal alcalin pour obtenir l'ester d'acide (S)-(-)-α-lipoïque et
c) la transformation de cet ester en acide (S)-(-)-α-lipoïque.
